# EUROPEAN PATENT SPECIFICATION

(11) **EP 0 663 464 B1**
(45) Date of publication and mention of the grant of the patent: **24.05.2000**
(21) Application number: 94307914.5
(22) Date of filing: 27.10.1994
(51) Int. Cl.: D04H 13/00

(54) **Liquid absorbing sheet material, method of manufacturing the liquid absorbing sheet material, method of manufacturing a sheet material and sanitary articles produced thereby**
Flüssigkeitsabsorbierender Vliesstoff, Verfahren zur Herstellung von dem Flüssigkeitsabsorbierenden Vliesstoff, Verfahren zur Herstellung eines Vliesstoffs und so hergestellte hygienische Artikel
Non-tissé en feuille absorbant les liquides, procédé pour la fabrication du non-tissé en feuille absorbant les liquides, procédé pour la fabrication d'un non-tissé en feuille et articles hygiéniques ainsi fabriquées

(30) Priority: 29.12.1993 JP 35312093; 29.12.1993 JP 35311993
(43) Date of publication of application: 19.07.1995
(73) Proprietor: UNI-CHARM CORPORATION, Kawanoe-shi Ehime-ken (JP)
(72) Inventor: Takamitsu, Igaue, Kawanoe-shi Ehime 799-01 (JP); Tsutomu, Kido, Kawanoe-shi Ehim 799-01 (JP); Ryohei, Sakakibara, Kobe-shi Hyogo 658 (JP); Masaharu, Sugie, Osaka-shi Osaka 534 (JP)
(74) Representative: Marlow, Nicholas Simon

(56) References cited:
- GB-A- 1 144 110
- GB-A- 2 190 111
- GB-A- 2 214 201

## Description

### FIELD OF THE INVENTION

This invention relates to liquid absorbing sheet material as covering material for a surface layer of sanitary articles such as a disposable diaper, a sanitary napkin, a surgical absorbing sheet, a method for producing thereof, a method for producing a sheet material, and sanitary articles produced by the above two kinds of material.

### BACKGROUND OF THE INVENTION

Generally, sanitary articles such as a disposable diaper and a sanitary napkin comprises liquid absorbing material, polyethylene sheet arranged on a reverse side of the liquid absorbing material for preventing liquid leak and covering material made of non-woven fabric, which covers the whole of the liquid absorbing material and the polyethylene sheet. The absorbing property of such sanitary articles greatly depends on the liquid permeability of the covering material. That is, there is an indispensable property for such covering material. It is most desirable that excreted body fluid passes through the covering material surface made of non-woven fabric immediately without staying thereon and reaches to the liquid absorbing material inside so that the covering material surface maintains always dry state so as not to give wet feeling to a user.

Heretofore, cellulose material has been employed as the above covering material, which does not meet with the above requirements. Replacing the cellulose material, polyester or polypropylene material has come into use. However, even though either polyester or polypropylene material is employed, it shows poor permeability, for example, excreted body fluid turns into water drops on the surface. To solve such a problem, such covering material as given liquid permeability by conducting surface active agent treatment with soaking treatment, spraying, baking or the like on the surface based upon non-woven fabric is currently employed. Such base non-woven fabric for the covering material can be obtained, for example, by preparing web for non-woven fabric material and sticking the web with adhesive. Subsequently, thus obtained non-woven fabric is formed into a sheet and wound up in a roll shape one by one. Thus, the covering material can be manufactured by employing non-woven fabric wound up in a roll one by one, withdrawing the non-woven fabric consecutively from the wound-up roll to be transported, conducting surface active agent on the non-woven fabric during the transportation and finally winding the non-woven fabric in another roll.

The first or the second excretion of body fluid at the initial stage can be permeated smoothly through the above non-woven woven fabric treated with the above surface active agent. However, the non-woven fabric has a drawback that permeability deteriorates with more excretion of body fluid since the surface active agent starts to flow out, resulting in deterioration of the permeability. Furthermore, non-woven fabric wherein ever fiber is treated with surface active agent has permeability, however, its surface becomes wet so as to make the user feel uncomfortable.

Still furthermore, the prepared non-woven fabric may be usually shaped in a sheet, which is usually wound up in a roll. Therefore, it is required to lower the transportation speed in changing rolls on a manufacturing line. Therefore, to improve productivity, the length of the non-woven fabric sheet for the one roll must be set up as long as possible, and such a demand has been requested. However, the thickness of the non-woven fabric in a roll shape is limited to be wound up in a roll, heretofore, how to increase the length of the non-woven fabric per one roll has been studied by lowering METSUKE volume as much as possible so as to thin the thickness. However, the lower limit of METSUKE volume is usually said to be about 20g/m². With lower METSUKE, resultant non-woven fabric becomes uneven, moreover, adhesive for joining often bleeds, which causes a problem that a range for application is narrowed.

### OBJECT OF THE INVENTION

GB-A-2190111 discloses an absorbent protective non-woven fabric comprising central layers of hydrophobic fibrous material, sandwiched between surface layers which have been rendered hydrophilic, the arrangement being such as to prevent aqueous liquid which impinges upon one surface from striking through to the other surface.

Accordingly it is an object of the invention to provide a liquid absorbing sheet material method of manufacturing the liquid absorbing sheet material, method of manufacturing a sheet material and sanitary articles produced by the above two kinds of material.

### DISCLOSURE OF THE INVENTION

The present invention is defined in the independent claims appended hereto, to which reference should now be made.

In order to accomplish the above object, the first gist of the present invention is related to liquid absorbing sheet material wherein fibrous body made of liquid conveyable material is scattered extending from a reverse side to a surface of a fabric tissue of a fabric base material. The second gist is related to liquid absorbing sheet material containing fabric base material and a second fabric bass material comprising heat fusible polymer laminated on one side of the fabric base material, wherein fibrous body made of liquid conveyable material is scattered extending from a reverse side to a surface in both fabric tissue comprising two-layer of the fabric base material and the second fabric base material. The third gist is a method of preparing liquid absorbing sheet material comprising transporting fabric base material in a shape of a sheet consecutively, forming a second fabric base material on the fabric base material by spraying heat fusible polymer on the fabric base material and spraying fibrous body comprising liquid conveyable material on the second fabric base material surface so as to have the liquid conveyable material invade in both tissue of the fabric base material and the second fabric base material. And the fourth gist is a method of preparing sheet material comprising transporting fabric base material in a shape of a sheet consecutively and forming a second fabric base material on the fabric base material by spraying heat fusible polymer on the fabric base material. Finally, the fifth gist is a sanitary article wherein the liquid absorbing sheet material defined in any of claims 1 to 4 is arranged on a reverse side of the liquid absorbing sheet material.

Namely, the present invention is related to liquid absorbing sheet material, wherein fibrous material comprising liquid conveyable material in the fabric tissue of the fabric base material is scattered extending from the reverse side to the surface. Alternately, the above single layer can be substituted with two-layer of a fabric base material and a second fabric base material comprising heat fusible polymer laminated on the one side of the fabric base material, wherein fibrous body made of liquid conveyable material is scattered as same as the above. Therefore, this liquid absorbing sheet material can introduce liquid to permeate from the surface to the reverse side, moreover, dry state on the surface can be maintained in absorbing moisture at several times without deterioration of permeability of moisture. Among such liquid absorbing sheet material, the second fabric base material in two-layer structure of the fabric base material and heat fusible polymer laminated on one side of the fabric base material can be obtained by steps comprising forming newly a second fabric base material layer on the fabric base material surface by spraying heat fusible polymer on the fabric base material surface during consecutive transportation of the sheet fabric base material and spraying fibrous body made of liquid conveyable material on the second fabric base material surface so as to have the liquid conveyable material invade in both tissue organization of the base fabric material and the second fabric base material. Furthermore, two-layer sheet material can be obtained by spraying heat fusible polymer on the fabric base material to form a newly second fabric base material layer on the fabric base material surface in the consecutive transportation process of the sheet fabric material of the above method. Therefore, thus obtained liquid absorbing sheet material or sheet material is thick and uniform. As a result, sheet fabric material in thin thickness as base can be employed. Hence, frequency of slowing down transportation speed due to roll shifts can be decreased, since the length per one roll becomes longer by winding the sheet at much times.

The present invention is now described in further detail.

The liquid absorbing sheet material in the present invention can be classified roughly into two modes. The first mode of the liquid absorbing sheet material, which is singly layer structure, can be obtained by employing fabric base material and liquid conveyable material reforming the fabric base material into permeable one. On the other hand, the second mode of the liquid absorbing sheet material, which comprises two layer of fabric base material and a second fabric base material, can be obtained by employing fabric base material, heat fusible polymer forming the second fabric base material and liquid conveyable material reforming the fabric base material into permeable one. In addition, liquid conveyable material means one that does not show solubility itself and does have hydrophilic property.

First, the first mode of liquid absorbing sheet material is described.

As the fabric base material employed for the first mode, there are no limitation and any known material can be employed according to the case, however, specifically, there are union fabric, non-woven fabric or the like. Among them, non-woven fabric is preferable. As the non-woven fabric, based upon the principle of the present invention, non-woven fabric whose METSUKE volume is low can be employed and is preferable. The non-woven fabric not more than 20g/m² for METSUKE volume, which is a limit to cause a various kinds of problems during manufacturing process in which prior non-woven fabric is employed. As the non-woven fabric, specifically, 20 to 60g/m² for METSUKE volume is preferable, 25 to 50g/m² is more preferable and 30 to 40g/m² is most preferable. That is, if METSUKE volume falls within the above limitation, fibrous body made of liquid conveyable material, as described later, can be invaded into fabric organization of fabric base material so as to disperse therein in good condition. Besides, the thickness of the fabric base material is set up appropriately in accordance with the application, however, generally, it is preferable to set up within 0.1 and 3mm.

As liquid conveyable material to reform the fabric base material into liquid conveyable one, heat fusible polymer is preferable. As the heat fusible polymer, there are polyolefine such as polypropylene (PP), polyethylene (PE), copolymer of PP and PE, styrene-isoprene block copolymer (SIS), styrene-butadiene block copolymer (SBS), hydrogenated SIS (SEPS), hydrogenated SBS (SEBS) and the like. These can be employed solely or in combination of two or more. Especially, the above SEPS and SEBS are preferable due to their thermal stability. For example, if water soluble polymer is employed as the liquid conveyable material, the polymer dissolves by contacting with moisture, which causes an unpreferable result of blocking on texture of the fabric base material.

As the liquid conveyable material, heat fusible polymer can be employed solely, however, it is preferable that surface active agent is mixed therein. More effective liquid conveyable property can be imparted to the fibrous body by mixing the surface active agent when forming fibrous body from heat fusible polymer. As the surface active agent, for example, nonionic surface active agent having one or two esters, which is fatty acid ester of polyethylene glycol or glycerine or nonionic surface active agent, which is block copolymer of polyethylene glycol and polypropylene glycol is employed preferably. The surface active agent is employed at the ratio of 0.1 to 20% by weight (abbreviated as % hereinafter), preferably at the ratio of 0.5 to 10% to the whole heat fusible polymer to be kneaded with the polymer at a high temperature.

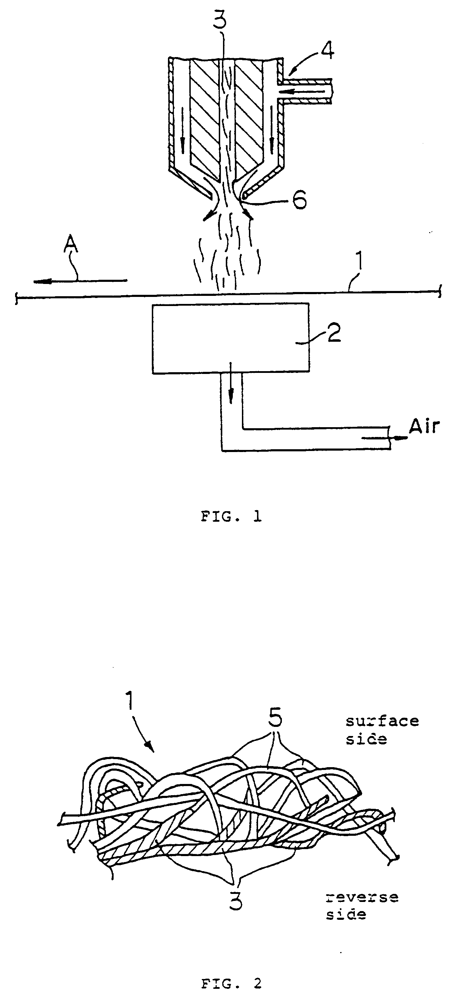

Secondly, a method for a liquid absorbing sheet material as the first mode is described. The liquid absorbing sheet material as the first mode can be manufactured, for example, in the following method. That is, the liquid absorbing sheet material can be prepared by melting the liquid conveyable material substantially composed of heat fusible polymer with heat and spraying it on the fabric base material surface. Specifically, as shown in figure 1, vacuum apparatus 2 is established under non-fabric woven 1, which goes in direction of an arrow A, so as to conduct absorbing in direction of an arrow, while heat fusible polymer 3 is sprayed heat fusible polymer (liquid conveyable material) over the surface of the non-fabric woven 1. At that time, air is sprayed the heat fusible polymer 3 from circumference immediately after being exhausted from an exit 6 inside an applicator 4 for spraying, the polymer 3 changes into fibrous state to be sprayed on the surface of the non-woven fabric 1 and invaded into the non-woven fabric 1. Thus a liquid absorbing sheet material can be manufactured.

Thus manufactured liquid absorbing sheet material, as shown in figure 2, has a structure wherein fibrous body converted from the heat fusible polymer 3 (liquid conveyable material) scatters from the surface to the reverse side among every one piece fiber 5 of non-woven fabric 1. The thickness of the fibrous body of converted from the heat fusible polymer 3 is set within about 0.3 to 3 denier.

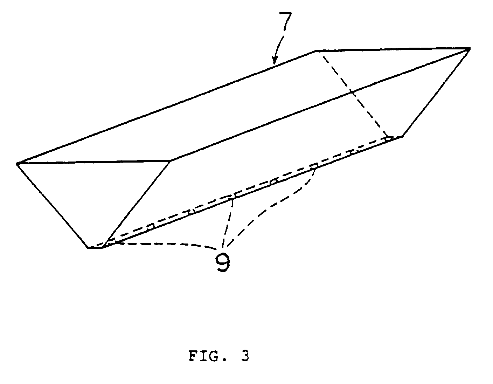

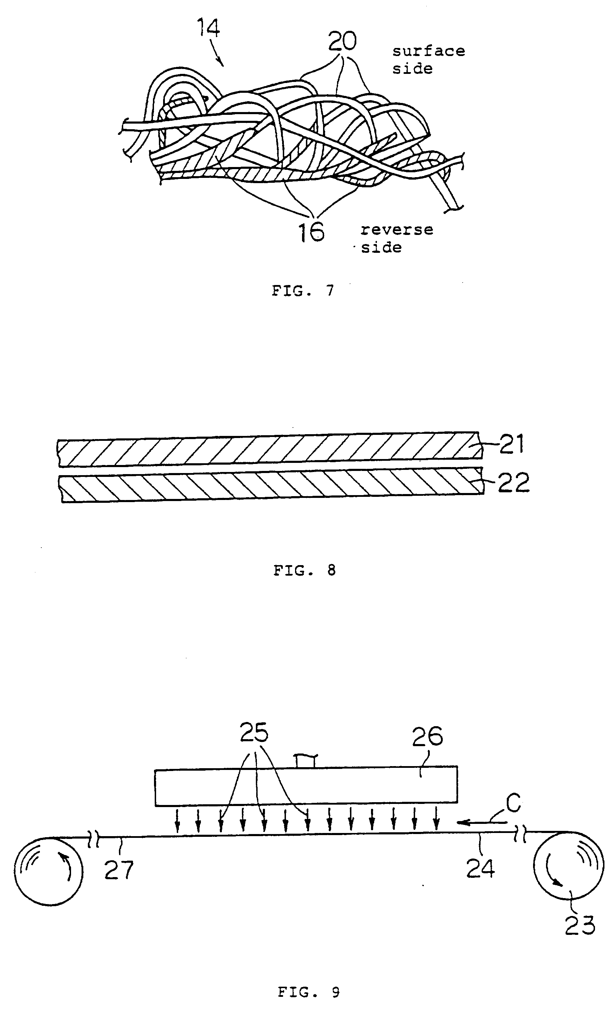

As method for the spraying, there is a method for spraying heat fusible polymer by a polymer spray applicator. As the applicator for spraying, there are hot melt spray, spiral spray and the like. Moreover, if a melt-blown applicator 7, as shown in figure 3, is employed, uniform spraying of the heat fusible polymer 3 on the fabric base material surface can be realized regardless of a line speed, which is preferable. In figure 3, 9 is an exit.

Next, the liquid absorbing sheet material of the second mode is described here.

The absorbing sheet material of the second mode, as mentioned before, is composed of two-layer structure of fabric base material and the second fabric base material and can be obtained by employing the fabric base material and the heat fusible polymer forming the second fabric base material, and the liquid conveyable material which conveys those two material into permeable ones.

As the fabric base material, the same fabric base material as that of the liquid absorbing sheet material in the above first mode can be employed.

In addition, as the heat fusible polymer forming the second fabric base material layer, there are a various kind of heat fusible polymer such as polyolefine of PP, PE or the like, copolymer of PP and PE, SIS, SBS, hydrogenated SIS (SEPS), hydrogenated SBS (SEBS) and the like, which are the same as those employed for the first mode. Among all, from the viewpoint of thermal stability, SEPS and SEBS are especially preferable.

As liquid conveyable material which converts the both fabric base material (fabric base material and the second fabric base material) into permeable ones, hydrophilic polymer is employed. The hydrophilic polymer can be obtained by melting heat fusible polymer and suitable active surface agent having a good compatibility therewith to be mixed. As the above heat fusible polymer, there are polyolefine such as PP, PE, copolymer of PP and PE, SIS, SBS, hydrogenerated SIS (SEPS), hydrogenated SBS (SEBS) and the like, which are the same as the heat fusible polymer. These are employed solely or in combination of two or more. In particular, the above SEPS and SEBS can be preferably employed due to their thermal stability. For example, water soluble polymer is not preferable as the liquid conveyable material because the polymer dissolves by contacting with moisture so as to cause blocking of the texture of the fabric base material.

Subsequently, as the surface active agent, which imparts hydrophilic property, to be melted and mixed to the heat fusible polymer, for example, there are nonionic surface active agent having one or two esters, which is fatty acid ester of polyethylene glycol or glycerine or nonionic surface active agent, which is block copolymer of polyethylene glycol and polypropylene glycol is employed preferably. The above surface active agent is set between 0.3 and 20% of the whole hydrophilic polymer (heat fusible polymer plus surface active agent), which is liquid conveyable material, preferably between 0.5 and 10%, and is kneaded with the heat fusible polymer, for example, at a high temperature. Thus, the hydrophilic polymer as the liquid conveyable material can be obtained.

Subsequently, a method of liquid absorbing sheet material as the second mode is described.

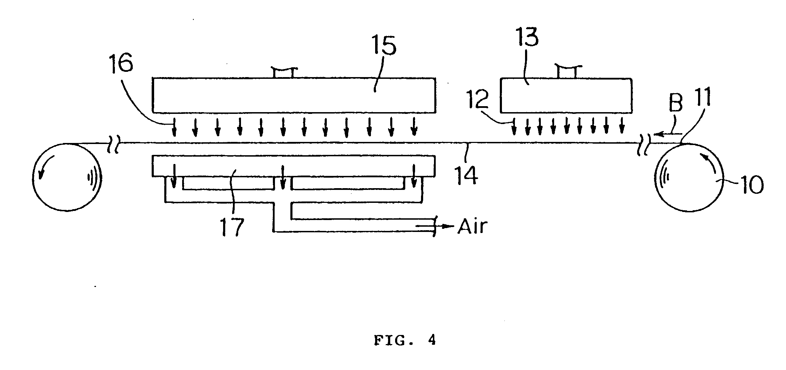

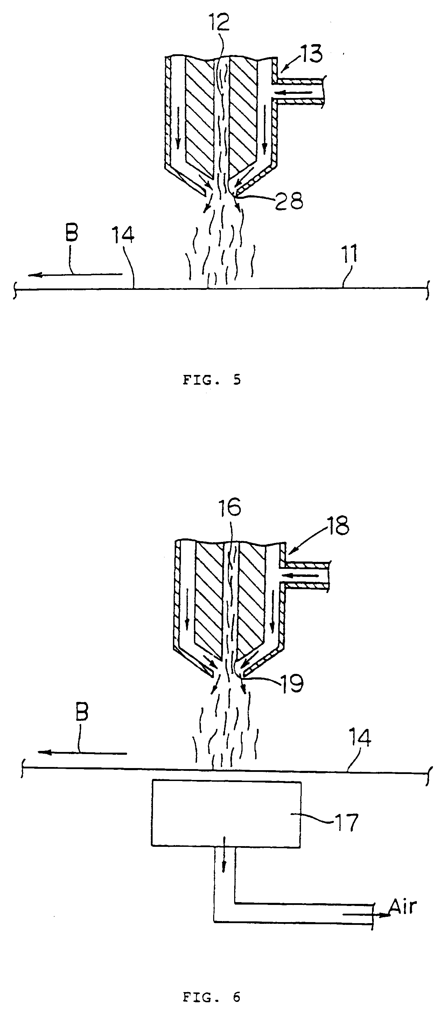

The liquid absorbing sheet material of the second mode can be prepared, for example, in the following method. That is, hydrophilic polymer (liquid conveyable material) is prepared by melting surface active agent so as to be mixed into heat fusible polymer. Then, as shown in figure 4, non-woven fabric sheet is transported consecutively in a direction of an arrow B from a roll 10 wherein non-woven fabric sheet is wound up. In this process, first thermoplastic polymer 12 is sprayed with a spraying apparatus 13 on the surface of non-woven fabric 11, which thereby forms a new second non-woven fabric layer, resulting in non-woven fabric 14 of a two-layer structure. In this way, non-woven fabric 14 of a two-layer structure for sheet material can be prepared (which corresponds to the method of preparing sheet material in the present invention). The method of this two-layer structure sheet material is described in detail. Heat fusible polymer 12 is sprayed over the surface of non-woven fabric sheet 11 which goes in direction of an arrow B. At that time, as shown in figure 5, air is blown to melted heat fusible polymer 12 from circumference of an exit 28 in a direction of an arrow immediately after charging out of an exit 28 inside an applicator 13 for blowing, so that the heat fusible polymer 12 is converted into fibrous state and sprayed over the non-woven fabric 11, which forms the second non-woven fabric layer.

Subsequently, the liquid absorbing sheet material can be manufactured by meltng the hydrophilic polymer 16 with heat and spraying over the surface of the two-layer structure non-woven fabric 14 (two-layer structure of non-woven fabric sheet 11 plus the second non-woven fabric layer). In detail, hydrophilic polymer 16 is sprayed over the surface of non-woven fabric 14, by absorbing the hydrophilic polymer by providing vacuum 17 beneath the non-woven fabric 14 of two-layer structure, which goes in a direction of an arrow B, at the same time. At that time, air is blown to the melted hydrophilic polymer 16, as shown in figure 6, from the circumference of the exit 19 in a direction of an arrow soon after the melted hydrophilic polymer is blown out of an exit 19 inside an applicator 18 for spraying. As a result, the hydrophilic polymer 16 is converted into fibrous body, which is blown over the surface of non-woven fabric 14 of two-layer structure and invade inside the non-woven fabric 14.

As a method of spraying of the heat fusible polymer 12 and hydrophilic polymer 16, there are a method of emitting the polymer with an applicator having polymer blower. As an applicator having polymer blower, there are hot melt spray, spiral spray and the like as same as those of the first mode for the liquid absorbing sheet material. As shown in figure 3, when employing melt blown applicator for non-woven fabric manufacturing, uniform spraying on the polymer can be realized regardless of the line speed, which is preferable.

Thus obtained liquid absorbing sheet material, as shown in figure 7, the fibrous body converted from hydrophilic polymer 16 is invaded and scattered into every one piece of fiber 20 of two-layer non-woven fabric 14, so as to pass through from the surface to the reverse side. The thickness of the hydrophilic polymer 16 converted into the fibrous body 3 is set within about 0.3 to 3 denier. In addition, it is preferable that the thickness of the second non-woven fabric layer formed with heat fusible polymer 12 is set within 0.15 to 3.0mm.

A sanitary article of the present invention, as shown in figure 8, can be obtained by arranging mat type water absorbing material 22 on the reverse side of liquid absorbing sheet material 21 obtained in the above method. Thickness of the mat type water absorbing material 22, is usually set within 5 to 10mm. The total thickness of the liquid absorbing sheet material 21 and mat type water absorbing material 22 is usually set within 5 to 13mm.

At that time, as both liquid absorbing sheet material 21 of the first mode and of the second mode, in case of imparting adhesive property to the liquid conveyable material of fibrous body scattered in the liquid absorbing sheet material 21, it is possible that not only permeability of liquid can be improved, but also liquid absorbing sheet material 21 and mat type water absorbing material 22 arranged on the reverse side thereof can be adhered.

As mat type water absorbing material 22, there are no limitation, for example, there is conventional known material employed. For example, there is a sheet obtained by forming high water absorbing polymer comprising pulp and sodium polyacrylate into a sheet state.

### EFFECT OF THE INVENTION

As mentioned hereinbefore, the present invention is related to liquid absorbing sheet material wherein fibrous body comprising liquid conveyable material extending from the reverse side to the surface in the fabric tissue of the fabric base material. Or, the present invention is related to liquid absorbing sheet material of two-layer structure comprising fabric base material and a second fabric base material made of heat fusible polymer, laminated on the fabric base material, wherein fibrous body of liquid conveyable material is scattered, instead of the single layer structure. Therefore, this liquid absorbing sheet material retains an excellent permeability even though liquid contacts and passes through thereto at several times, resulting in a dry state of the surface of the sheet material. Liquid on the surface of liquid absorbing sheet material can be led to the fibrous body without staying on the surface and be reached to be absorbed with the absorbing sheet material arranged of the reverse side. In addition, since the fibrous body scattered in the above liquid absorbing sheet material has an adhesive effect, the sheet material and mat type water absorbing material arranged on the reverse side can be adhered when manufacturing a sanitary article by arranging the maty type absorbing material on the liquid absorbing sheet material. When adhering these two materials, another troublesome process such as installing an adhesive layer between them can be abbreviated, resulting in cost decrease and simplification of a manufacturing process and the structure of the product. Among such liquid absorbing shee materials, two-layer liquid absorbing sheet material comprising fabric base material and a second fabric base material made of heat fusible polymer, laminated on one side of the fabric base material can be manufactured by forming the second new fabric base material with sprayed heat fusible polymer on the surface of the fabric base material during consecutive transportation process. Subsequently, the fibrous body comprising liquid conveyable material is sprayed thereon so that the liquid conveyable material is invaded into the both tissue organization of the fabric base material and the second fabric base material. Therefore, the thickness of thus obtained liquid absorbing sheet material or two-layer structure sheet material is thick and uniform. Concomitantly, thin fabric base material can be employed as base. Therefore, for example, by increasing the turning number on a roll and making the length of a roll per one longer, productivity can be improved due to decrease of the number of times to lower the transportation speed in roll shifting. The sanitary articles employing such liquid absorbing sheet material are most suitable for materials of, for example, a disposable diaper, a sanitary napkin, surgical absorbing sheet or the like.

### BRIEF DESCRIPTION OF THE DRAWINGS

Fig. 1 illustrates one embodiment of manufacturing processes of liquid absorbing sheet material in the present invention,
Fig. 2 is a diagrammatic illustration of a structure of liquid absorbing sheet material in the present invention,
Fig. 3 is a perspective view of melt-blown applicator,
Fig. 4 illustrates another embodiment of manufacturing process of liquid absorbing sheet material in the present invention,
Fig. 5 illustrates how an applicator emits heat fusible polymer in a manufacturing process of two-layer structure sheet material,
Fig. 6 illustrates how an applicator emits heat fusible polymer,
Fig. 7 is a diagrammatic illustration of another embodiment of liquid absorbing sheet material,
Fig. 8 shows a sectional view of a sanitary article of the present invention,
Fig. 9 illustrates a manufacturing process of sheet material of the present invention,
Fig. 10 illustrates how polymer exit emits heat fusible polymer and
Fig. 11 is a sectional view of the above sheet material.

The following examples and comparative examples are further illustrative of the invention.
(1) Liquid absorbing sheet material of the first mode was manufactured.

### EXAMPLES 1 to 9

First of all, polymer was prepared by melting and kneading each component shown in the following table 1. On the other hand, non-woven fabric of METSUKE volume at 20g/m² was prepared. Subsequently, as shown in figure 1, the above polymer 3 was sprayed over the surface of non-woven fabric 1, which goes in a direction of an arrow A, as absorbing in a direction of an arrow by a vacuum apparatus 2 installed under the non-woven fabric 1. Thus liquid absorbing sheet material was prepared.

**TABLE 1**

| | EXAMPLE | | | | | | | | |
|---|---|---|---|---|---|---|---|---|---|
| | 1 | 2 | 3 | 4 | 5 | 6 | 7 | 8 | 9 |
| | | | | | | | | | |

| POLYOLEFINE RESIN *1 | | | | | | | | | |
|---|---|---|---|---|---|---|---|---|---|
| A | 80 | - | - | 50 | - | - | - | - | - |
| B | - | 90 | - | - | 40 | 25 | - | - | - |
| C | - | - | 99 | 30 | 40 | 70 | - | - | - |
| D | - | - | - | - | - | - | 95 | - | 97 |
| E | - | - | - | - | - | - | - | 85 | - |

| SURFACE ACTIVE AGENT | | | | | | | | | |
|---|---|---|---|---|---|---|---|---|---|
| A *2 | 20 | 10 | 1 | 15 | 20 | 5 | 5 | 15 | - |
| B *3 | - | - | - | - | - | - | - | - | 3 |
| ANTIOXIDANT *4 | 0.5 | 0.5 | 0.5 | 0.5 | 0.5 | 0.5 | 0.5 | 0.5 | 0.5 |

| | | | | | | | | | |
|---|---|---|---|---|---|---|---|---|---|
| (NB) *1 : polypropylene-polyethylene copolymer | | | | | | | | | |
| *2 : polyethylene glycol-fatty acid ester | | | | | | | | | |
| *3 : glycerine fatty acid ester | | | | | | | | | |
| *4 : phenol | | | | | | | | | |

### COMPARATIVE EXAMPLES 1 to 6

Instead of the above heat fusible polymer, water soluble polymer shown in the following table 2 was employed. In the same way except for that, liquid absorbing sheet material was manufactured.

**TABLE 2**

| | COMPARATIVE EXAMPLES | | | | | |
|---|---|---|---|---|---|---|
| | 1 | 2 | 3 | 4 | 5 | 6 |
| | | | | | | |

| WATER SOLUBLE POLYMER | | | | | | |
|---|---|---|---|---|---|---|
| A *1 | 100 | - | - | - | - | - |
| B *2 | - | 100 | - | - | - | - |
| C *3 | - | - | 100 | - | - | - |
| D *4 | - | - | - | 100 | - | - |
| E *5 | - | - | - | - | 100 | - |
| F *6 | - | - | - | - | - | 100 |
| ANTIOXIDANT *7 | 0.5 | 0.5 | 0.5 | 0.5 | 0.5 | 0.5 |

| | | | | | | |
|---|---|---|---|---|---|---|
| (NB) *1 : polyethylene glycol | | | | | | |
| *2 : polyethylene glycol | | | | | | |
| *3 : polyethylene glycol | | | | | | |
| *4 : polyvinyl pyrrolidone-vinyl acetate copolymer | | | | | | |
| *5 : ethylene oxide-propylene oxide copolymer | | | | | | |
| *6 : ethylene oxide-propylene oxide copolymer | | | | | | |
| *7 : phenol | | | | | | |

### CONVENTIONAL EXAMPLE 1

Non-woven fabric of METSUKE 20g/m² was prepared. Nonionic surface active agent (Nonipole 500: Sanyokasei-sha product) was sprayed thereon. Thus nonionic surface active agent was produced.

Artificial urine was dropped on thus obtained each example and comparative example so as to measure each contact angle. The results were shown in the following tables 3 to 6.

In addition, when measuring the contact angle, each liquid absorbing sheet material, which had been left untreated, in water of 20°C, in water of 40°C and in water of 60 °C for a week respectively.

Furthermore, evaluating test of liquid absorbing property was measured as follows. First, 50cc of blue-colour artificial urine was dropped on each non-woven fabric so as to measure the time taken to absorb the urine (initial value). Subsequently, five minutes later, another 50cc of blue-colour artificial urine was dropped in the same as the above so as to measure the time taken to absorb the urine by visual examination. Further, another five minutes later 50cc of blue-colour artificial urine was dropped to measure the absorbing time. Thus, the change of the time was measured on each non-woven fabric by this repetition. The results were shown in the following tables 3 to 6. After the above measurement, panelists valued each feel by touching each surface of non-woven fabric. ○ means dry feeling while X means wet feeling in tables 3 to 6.

**TABLE 3**

| | EXAMPLES | | | | |
|---|---|---|---|---|---|
| | 1 | 2 | 3 | 4 | 5 |
| | | | | | |

| CONTACT ANGLE ( ° ) | | | | | |
|---|---|---|---|---|---|
| untreated | 0 | 0 | 0 | 0 | 0 |
| left in water (20 °C) for 1 week | 0 | 0 | 0 | 0 | 0 |
| left in water (40 °C) for 1 week | 0 | 0 | 5 | 0 | 0 |
| left in water (60 °C) for 1 week | 0 | 0 | 7 | 0 | 0 |

| LIQUID ABSORBING PROPERTY (seconds) | | | | | |
|---|---|---|---|---|---|
| at the initial stage | < 1 | < 1 | < 1 | < 1 | < 1 |
| 50cc of artificial urine was dropped | < 1 | < 1 | < 1 | < 1 | < 1 |
| 100cc of artificial urine was dropped | < 1 | < 1 | < 1 | < 1 | < 1 |
| FEEL OF SURFACE | ○ | ○ | ○ | ○ | ○ |

**TABLE 4**

| | EXAMPLES | | | |
|---|---|---|---|---|
| | 6 | 7 | 8 | 9 |
| | | | | |

| CONTACT ANGLE ( ° ) | | | | |
|---|---|---|---|---|
| untreated | 0 | 0 | 0 | 0 |
| left in water (20 °C) for 1 week | 0 | 0 | 0 | 0 |
| left in water (40 °C) for 1 week | 3 | 3 | 5 | 5 |
| left in water (60 °C) for 1 week | 3 | 3 | 7 | 5 |

| LIQUID ABSORBING PROPERTY (seconds) | | | | |
|---|---|---|---|---|
| at the initial stage | < 1 | < 1 | < 1 | < 1 |
| 50cc of artificial urine was dropped | < 1 | < 1 | < 1 | < 1 |
| 100cc of artificial urine was dropped | < 1 | < 1 | < 1 | < 1 |
| FEEL OF SURFACE | ○ | ○ | ○ | ○ |

**TABLE 5**

| | COMPARATIVE EXAMPLES | | | | |
|---|---|---|---|---|---|
| | 1 | 2 | 3 | 4 | 5 |
| | | | | | |

| CONTACT ANGLE ( ° ) | | | | | |
|---|---|---|---|---|---|
| untreated | 15 | 20 | 10 | 30 | 40 |
| left in water (20 °C) for 1 week | dissolved | | | | |
| left in water (40 °C) for 1 week | - | - | - | - | - |
| left in water (60 °C) for 1 week | - | - | - | - | - |

| LIQUID ABSORBING PROPERTY (seconds) | | | | | |
|---|---|---|---|---|---|
| at the initial stage | < 1 | < 1 | < 1 | < 1 | < 1 |
| 50cc of artificial urine was dropped | > 30 | > 30 | > 30 | > 30 | > 30 |
| 100cc of artificial urine was dropped | > 30 | > 30 | > 30 | > 30 | > 30 |
| FEEL OF SURFACE | X | X | X | X | X |

**TABLE 6**

| | COMPARATIVE EXAMPLE 6 | CONVENTIONAL EXAMPLE 1 |
|---|---|---|
| | | |

| CONTACT ANGLE ( ° ) | | |
|---|---|---|
| untreated | 40 | 30 |
| left in water (20 °C) for 1 week | dissolved | 85 |
| left in water (40 °C) for 1 week | - | 90 |
| left in water (60 °C) for 1 week | - | 90 |

| LIQUID ABSORBING PROPERTY (seconds) | | |
|---|---|---|
| at the initial stage | < 1 | < 1 |
| 50cc of artificial urine was dropped | > 30 | > 30 |
| 100cc of artificial urine was dropped | > 30 | > 30 |
| FEEL OF SURFACE | X | ○ |

As clear from the above tables 3 to 6, water soluble polymer dissolved in COMPARATIVE EXAMPLES 1 to 6, so as to stuff the texture. In addition, although samples absorbed artificial urine less than 1 second at the initial stage of the test for liquid absorbing property, it took not less than 30 seconds due to deterioration of liquid absorbing property with repeated dropping of artificial urine. It is found out that even untreated samples of COMPARATIVE EXAMPLES were large in contact angles and had poor liquid absorbing property. On the other hand, compared with COMPARATIVE EXAMPLES, EXAMPLES were very small in contact angles and no deterioration of absorbing time was shown not only at the initial stage, but also at twice dropping of artificial urine in which the time of absorbing was less than 1 second. In addition, feel of surface in all EXAMPLES was dry.
(2) Secondly, sheet material as base for the liquid absorbing sheet material in the second mode was prepared.

First of all, prior to EXAMPLES, five compounds for heat fusible polymer were prepared as shown in the following table 7.

**TABLE 7**

| COMPOUNDS HEAT FUSIBLE POLYMER | |
|---|---|
| a | hydrogenated styrene-butadiene block copolymer (SEBS) |
| b | hydrogenated styrene-isoprene block copolymer (SEPS) |
| c | copolymer of polypropylene (PP) and polyethylene (PE) |
| d | polyethylene (PE) |
| e | polypropylene (PP) |

### EXAMPLE 10

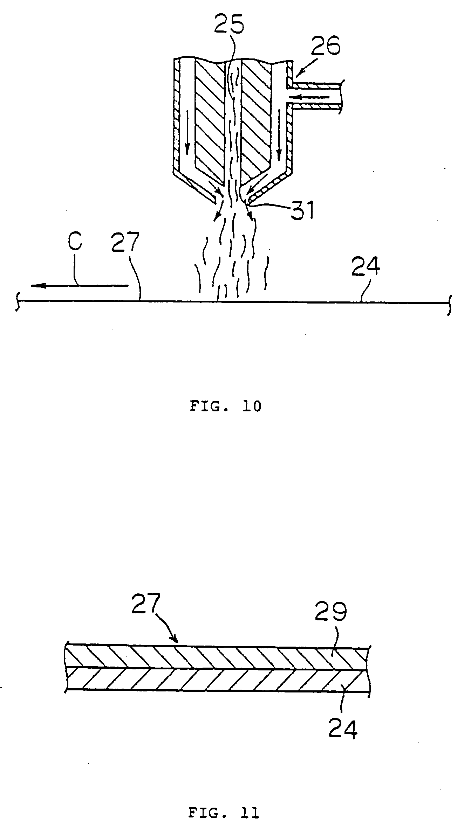

A roll wherein non-woven fabric sheet at 15g/m² for METSUKE volume was prepared. Subsequently, as shown in figure 9, the non-woven fabric 24 was drawn out from the roll 23 so as to be transported consecutively in a direction of an arrow C. During transportation, the above heat fusible polymer 25 (heat fusible polymer a), which was melted and converted into fibrous body, was sprayed on the surface of non-woven fabric 24 so as to form the second non-woven fabric layer. In detail, as shown in figure 10, air was blown on the heat fusible polymer 25 in a direction of an arrow from the circumference of the exit 31 soon after being emitted from an exit 31 of polymer emitter (an applicator for spraying) 26, so that the heat fusible polymer 25 was converted into fibrous body, which was sprayed on non-woven fabric 24, resulting in the second non-woven fabric layer. Melt blown applicator shown in figure 3 was employed for spraying of the heat fusible polymer 25. Thus, as shown in figure 11, two-layer structure sheet material 27, wherein the second non-woven fabric layer 29 comprising heat fusible polymer 25 was laminated, was prepared.

### EXAMPLE 11

Heat fusible polymer b of the table 7 was employed as heat fusible polymer. Except for that, sheet material was prepared in the same way as EXAMPLE 10.

### EXAMPLE 12

Heat fusible polymer c of the table 7 was employed as heat fusible polymer. Except for that, sheet material was prepared in the same way as EXAMPLE 10.

### EXAMPLE 13

A mixture of heat fusible polymer a and c (mixing ratio (by weight); a:c=5:3) of the table 7 was employed as heat fusible polymer. Except for that, sheet material was prepared in the same way as EXAMPLE 10.

### EXAMPLE 14

A mixture of heat fusible polymer b and c (mixing ratio (by weight); b:c=1:1) of the table 7 was employed as heat fusible polymer. Except for that, sheet material was prepared in the same way as EXAMPLE 10.

### EXAMPLE 15

A mixture of heat fusible polymer b and c (mixing ratio (by weight); b:c=25:70) of the table 7 was employed as heat fusible polymer. Except for that, sheet material was prepared in the same way as EXAMPLE 10.

### EXAMPLE 16

Heat fusible polymer d of the table 7 was employed as heat fusible polymer. Except for that, sheet material was prepared in the same way as EXAMPLE 10.

### EXAMPLE 17

Heat fusible polymer e of the table 7 was employed as heat fusible polymer. Except for that, sheet material was prepared in the same way as EXAMPLE 10.

Thus obtained sheet material of EXAMPLES 10 to 17 and untreated non-woven fabric on the market (METSUKE volume 12g/m³)(conventional example 2) were compared in each thickness. In addition, thick non-woven fabric wound on a roll on the market (METSUKE volume 25g/m²) (conventional example 3) was compared in thickness. The results are shown in the following tables 8 and 9.

**TABLE 8**

| EXAMPLES | | | | | | | |
|---|---|---|---|---|---|---|---|
| 10 | 11 | 12 | 13 | 14 | 15 | 16 | 17 |
| | | | | | | | |

| THICKNESS OF SHEET MATERIAL (mm) | | | | | | | |
|---|---|---|---|---|---|---|---|
| 0.15 | 0.20 | 0.22 | 0.17 | 0.17 | 0.20 | 0.20 | 0.18 |

**TABLE 9**

| CONVENTIONAL EXAMPLES | |
|---|---|
| 2 | 3 |
| | |

| THICKNESS OF SHEET MATERIAL (mm) | |
|---|---|
| 0.10 | 0.17 |

As clear from the results of tables 8 and 9, in spite that thickness of non-woven fabric is of METSUKE volume 15g/m² at the initial material, thickness became thick and surface was uniform. That is, thickness more than that of non-woven fabric in conventional example 3 was obtained.
(3) Then, liquid absorbing sheet material of the second mode was manufactured.

### EXAMPLES 18 to 25

First of all, hydrophilic polymer was produced by melting each component shown in the following table 10 to be kneaded. On the other hand, a roll wherein non-woven fabric sheet of METSUKE volume 15g/m² is wound was prepared. Subsequently, as shown in figure 4, non-woven fabric sheet 11 was drawn consecutively out of the roll 10 in a direction of an arrow B. During transportation, polypropylene-polyethylene copolymer 12 as heat fusible polymer was sprayed on the surface of non-woven fabric 11 from an applicator 13 having a polymer blower so as to form the second non-woven fabric layer 14. Then, the hydrophilic polymer 16 was melted with heat and at the same time sprayed over the surface of the two-layer structure non-woven fabric 14 (two-layer structure of non-woven fabric sheet layer 11 and the second non-woven fabric layer) from polymer emitter 15. At the same time, absorbing was conducted in a direction of an arrow by a vacuum device 17 established under two-layer non-woven fabric 14. Thus, liquid absorbing sheet material was produced. Thus obtained every liquid absorbing sheet material were thick in thickness and had uniform surface. From observation by electron microscope, shown in figure 7, it is found out that hydrophilic polymer 16, which is converted into fibrous body, was invaded among every fiber 20 of two-layer non-woven fabrics 14 from one side to the other side.

**TABLE 10**

| | | EXAMPLES | | | | | | | |
|---|---|---|---|---|---|---|---|---|---|
| | | 18 | 19 | 20 | 21 | 22 | 23 | 24 | 25 |
| | | | | | | | | | |

| POLYOLEFINE RESIN *1 | | | | | | | | | |
|---|---|---|---|---|---|---|---|---|---|
| A | | 80 | - | - | 50 | - | - | - | - |
| B | | - | 90 | - | - | 40 | 25 | - | - |
| C | | - | - | 99 | 30 | 40 | 70 | - | - |
| D | | - | - | - | - | - | - | 95 | - |
| E | | - | - | - | - | - | - | - | 85 |
| SURFACE ACTIVE AGENT *2 | | 20 | 10 | 1 | 20 | 20 | 5 | 5 | 15 |
| ANTIOXIDANT *3 | 0.5 | 0.5 | 0.5 | 0.5 | 0.5 | 0.5 | 0.5 | 0.5 | 0.5 |

| | | | | | | | | | |
|---|---|---|---|---|---|---|---|---|---|
| (NB) *1 : polypropylene-polyethylene copolymer | | | | | | | | | |
| *2 : polyethylene glycol-fatty acid ester | | | | | | | | | |
| *3 : phenol | | | | | | | | | |

### CONVENTIONAL EXAMPLE 4

Non-woven fabric of METSUKE volume 15g/m² was prepared. Nonionic surface active agent (Nonipole 500, Sanyokasei-sha product) was sprayed on the surface of the fabric. Thus liquid absorbing sheet material treated with nonionic surface active agent was produced.

Thus obtained each examples and conventional example 4 were measured in their contact angles by dropping artificial urine. The results were shown in the following tables 11 and 12. In addition, when measuring contact angles, each liquid absorbing sheet material, which had been left untreated, in water of 20 °C, 40°C and 60 °C for a week.

Further, evaluation test of liquid absorbing property was conducted. First, the time taken to absorb 50cc of blue-color artificial urine dropped on each non-woven fabric was measured (initial evaluation). Subsequently, five minutes later, another 50cc of blue-colour artificial urine was dropped in the same as the above so as to measure the time taken to absorb the urine by visual examination. Further, five minutes later 50cc of blue-colour artificial urine was dropped to measure the time for absorbing. Thus, the change of the time was measured on each non-woven fabric by this repetition. The results were shown in the following tables 11 to 12. After the above measurement, panelists valued each feel by touching each surface of non-woven fabric. ○ means dry feeling while X means wet feeling in tables 11 to 12.

**TABLE 11**

| | EXAMPLES | | | | |
|---|---|---|---|---|---|
| | 18 | 19 | 20 | 21 | 22 |
| | | | | | |

| CONTACT ANGLE ( ° ) | | | | | |
|---|---|---|---|---|---|
| untreated | 0 | 0 | 0 | 0 | 0 |
| left in water (20 °C) for 1 week | 0 | 0 | 0 | 0 | 0 |
| left in water (40 °C) for 1 week | 0 | 0 | 5 | 0 | 0 |
| left in water (60 °C) for 1 week | 0 | 0 | 7 | 0 | 0 |

| LIQUID ABSORBING PROPERTY (seconds) | | | | | |
|---|---|---|---|---|---|
| at the initial stage | < 1 | < 1 | < 1 | < 1 | < 1 |
| 50cc of artificial urine was dropped | < 1 | < 1 | < 1 | < 1 | < 1 |
| 100cc of artificial urine was dropped | < 1 | < 1 | < 1 | < 1 | < 1 |
| FEEL OF SURFACE | ○ | ○ | ○ | ○ | ○ |
| THICKNESS OF WHOLE SHEET MATERIAL (mm) | 0.17 | 0.22 | 0.25 | 0.20 | 0.22 |

**TABLE 12**

| | EXAMPLES | | CONVENTIONAL EXAMPLE | |
|---|---|---|---|---|
| | 23 | 24 | 25 | 4 |
| | | | | |

| CONTACT ANGLE ( ° ) | | | | |
|---|---|---|---|---|
| untreated | 0 | 0 | 0 | 30 |
| left in water (20 °C) for 1 week | 0 | 0 | 0 | 85 |
| left in water (40 °C) for 1 week | 3 | 3 | 5 | 90 |
| left in water (60 °C) for 1 week | 3 | 3 | 7 | 90 |

| LIQUID ABSORBING PROPERTY (seconds) | | | | |
|---|---|---|---|---|
| at the initial stage | < 1 | < 1 | < 1 | < 1 |
| 50cc of artificial urine was dropped | < 1 | < 1 | < 1 | > 30 |
| 100cc of artificial urine was dropped | < 1 | < 1 | < 1 | > 30 |
| FEEL OF SURFACE | ○ | ○ | ○ | X |
| THICKNESS OF WHOLE SHEET MATERIAL (mm) | 0.23 | 0.23 | 0.20 | 0.10 |

As clear from the above tables 11 and 12, although samples absorbed artificial urine less than 1 second at the initial stage, it took not less than 30 seconds due to deterioration of liquid absorbing property with repeated dropping of artificial urine. It is found out that even untreated samples of CONVENTIONAL EXAMPLE was large in contact angles and had poor liquid absorbing property. On the other hand, compared with CONVENTIONAL EXAMPLE 4, each EXAMPLES were very small in contact angles and no deterioration of absorbing time was shown not only at the initial stage, but also at twice dropping of artificial urine in which the time of absorbing was less than 1 second. In addition, feel of surface in all EXAMPLES was dry. Further, in spite that non-woven fabric at low METSUKE volume of 15g/m² was employed, finally thick thickness in the same level as the case which employed thick METSUKE volume.

## Claims

1. A liquid absorbent sheet comprising a first layer of non-woven fabric base material throughout which are distributed liquid-conveyable fibres which extend through the layer of non-woven fabric base material from a first side to a second side of the sheet.

2. A sheet according to claim 1 comprising a second layer of fabric base material of heat-fusible polymer laminated on one side of the first layer, wherein the liquid-conveyable fibres extend through both the first and second layers from the first side to the second side of the sheet.

3. A sheet according to claim 1 or claim 2 wherein the liquid-conveyable fibres are of heat-fusible polymer containing surface active agent.

4. A sheet according to claim 2 or claim 3 wherein the heat-fusible polymer is at least one selected from the group consisting of polypropylene, polyethylene, copolymer of polypropylene and polyethylene, styrene-isoprene block copolymer, styrene-butadiene block copolymer, hydrogenated styrene-isoprene block copolymer and hydrogenated styrene-butadiene block copolymer.

5. A method of preparing a liquid absorbent sheet, the method comprising transporting a first layer of non-woven fabric base material in the form of a sheet, forming on the first layer a second layer of non-woven fabric base material by spraying heat-fusible polymer on the surface of the first layer, and spraying fibrous liquid-conveyable material on the second layer so that the liquid-conveyable fibres invade both the first and second layers, becoming distributed therein and extending from a first to a second side of the sheet.

6. A method according to claim 5 wherein the liquid-conveyable material is heat-fusible polymer containing surface active agent.

7. A method according to claim 5 or claim 6 wherein the heat-fusible polymer is at least one selected from a group consisting of polypropylene, polyethylene, copolymer of polypropylene and polyethylene, styrene-isoprene block copolymer, styrene-butadiene block copolymer, hydrogenated styrene-isoprene block copolymer and hydrogenated styrene-butadiene block copolymer.

8. A method of preparing a liquid absorbent sheet, the method comprising transporting a fist layer of fabric base material in the form of a sheet, forming on the first layer a second layer of fabric base material by spraying heat-fusible polymer on the surface of the first layer.

9. A method according to claim 8 wherein the heat-fusible polymer is at least one selected from a group consisting of polypropylene, polyethylene, copolymer of polypropylene and polyethylene, styrene-isoprene block copolymer, styrene-butadiene block copolymer, hydrogenated styrene-isoprene block copolymer and hydrogenated styrene-butadiene block copolymer.

10. A sanitary article comprising a liquid absorbent sheet according to any of claims 1 to 4, on one side of which is arranged a mat of liquid absorbent sheet material.

## Patentansprüche

1. Eine flüssigkeitsabsorbierende Lage, die eine erste Schicht von Vliesgrundmaterial umfasst, in welchem flüssig transportfähige Fasern verteilt sind, die sich durch die Schicht von Vliesgrundmaterial von einer ersten Seite der Lage bis zu ihrer zweiten Seite ausdehnen.

2. Eine Lage nach Anspruch 1, die eine zweite Schicht von Gewebegrundmaterial aus wärmeschmelzbarem Polymer umfasst, mit dem eine Seite der ersten Schicht beschichtet ist, in welcher sich die flüssig transportfähigen Fasern sowohl durch die erste als auch die zweite Schicht von der ersten bis zur zweiten Seite der Lage ausdehnen.

3. Eine Lage nach Anspruch 1 oder Anspruch 2, in der die flüssig transportfähigen Fasern aus wärmeschmelzbarem, tensidhaltigem Polymer sind.

4. Eine Lage nach Anspruch 2 oder Anspruch 3 mit mindestens einem wärmeschmelzbarem Polymer, das aus der Gruppe gewählt wird, die Polypropylen, Polyäthylen, Kopolymer von Polypropylen und Polyäthylen, Styrol-Isopren-Blockkopolymer, Styrol-Butadien-Blockkopolymer, hydriertes Styrol-Isopren-Blockkopolymer und hydriertes Styrol-Butadien-Blockkopolymer umfasst.

5. Ein Verfahren zur Vorbereitung einer flüssigkeitsabsorbierenden Lage, das den Transport einer ersten Schicht von Vliesgrundmaterial in Gestalt einer Lage, die Bildung einer zweiten Schicht von Vliesgrundmaterial auf der ersten Schicht durch Besprühen ihrer Oberfläche mit wärmeschmelzbarem Polymer und das Besprühen der zweiten Schicht mit faserigem, flüssig transportfähigem Material umfasst, so dass die flüssig transportfähigen Fasern sowohl die erste als auch die zweite Schicht durchdringen, darin verteilt werden und sich von einer ersten bis zu einer zweiten Seite der Lage ausdehnen.

6. Ein Verfahren nach Anspruch 5, bei dem das flüssig transportfähige Material ein wärmeschmelzbares, tensidhaltiges Polymer ist.

7. Ein Verfahren nach Anspruch 5 oder Anspruch 6, bei dem mindestens ein wärmeschmelzbares Polymer aus einer Gruppe gewählt wird, die Polypropylen, Polyäthylen, Kopolymer von Polypropylen und Polyäthylen, Styrol-Isopren-Blockkopolymer, Styrol-Butadien-Blockkopolymer, hydriertes Styrol-Isopren-Blockkopolymer und hydriertes Styrol-Butadien-Blockkopolymer umfasst.

8. Ein Verfahren zur Vorbereitung einer flüssigkeitsabsorbierenden Lage, das den Transport einer ersten Schicht von Gewebegrundmaterial in Gestalt einer Lage sowie die Bildung einer zweiten Schicht von Gewebegrundmaterial auf der ersten Schicht umfasst, indem wärmeschmelzbares Polymer auf die Oberfläche der ersten Schicht gesprüht wird.

9. Ein Verfahren nach Anspruch 8, bei dem mindestens ein wärmeschmelzbares Polymer aus einer Gruppe gewählt wird, die Polypropylen, Polyäthylen, Kopolymer von Polypropylen und Polyäthylen, Styrol-Isopren-Blockkopolymer, Styrol-Butadien-Blockkopolymer, hydriertes Styrol-Isopren-Blockkopolymer und hydriertes Styrol-Butadien-Blockkopolymer umfasst.

10. Ein hygienischer Artikel, der eine flüssigkeitsabsorbierende Lage nach einem der Ansprüche 1 bis 4 umfasst, die auf einer Seite mit einer Matte von flüssigkeitsabsorbierendem Vliesstoff versehen ist.

## Revendications

1. Feuille absorbant les liquides comprenant une première couche de matière de base de tissu non tissé à travers laquelle sont distribuées des fibres d'acheminement de liquides qui s'étendent à travers la couche de matière de base de tissu non tissé depuis un premier côté jusqu'à un deuxième côté de la feuille.

2. Feuille selon la revendication 1, comprenant une deuxième couche de matière de base de tissu de polymère thermofusible appliquée sur un côté de la première couche, dans laquelle les fibres d'acheminement de liquides s'étendent à travers les deux première et deuxième couches depuis un premier côté jusqu'à un deuxième côté de la feuille.

3. Feuille selon la revendication 1 ou la revendication 2, dans laquelle les fibres d'acheminement de liquides sont en polymère thermofusible contenant un agent actif de surface.

4. Feuille selon la revendication 2 ou la revendication 3, dans laquelle le polymère thermofusible est au moins un polymère sélectionné dans le groupe consistant en polypropylène, polyéthylène, copolymère de polypropylène et de polyéthylène, copolymère de blocs styrène-isoprène, copolymère en masse de styrène-butadiène, copolymère en masse de styrène-isoprène hydrogéné et copolymère en masse de styrène-butadiène hydrogéné.

5. Procédé de préparation d'une feuille absorbant les liquides, le procédé comprenant le transport d'une première couche de matière de base de tissu non tissé sous forme de feuille, la formation sur la première couche d'une deuxième couche d'une matière de base de tissu non tissé en vaporisant un polymère thermofusible sur la surface de la première couche, et la vaporisation d'une matière d'acheminement de liquides fibreuse sur la deuxième couche de telle sorte que les fibres d'acheminement de liquide envahissent à la fois les première et deuxième couches, se retrouvant distribuées dans celles-ci et s'étendant depuis un premier jusqu'à un deuxième côté de la feuille.

6. Procédé selon la revendication 5, dans lequel la matière d'acheminement de liquide est un polymère thermofusible contenant un agent actif de surface.

7. Procédé selon la revendication 5 ou la revendication 6, dans lequel le polymère thermofusible est au moins un polymère sélectionné dans un groupe consistant en polypropylène, polyéthylène, copolymère de polypropylène et de polyéthylène, copolymère en masse de styrène-isoprène, copolymère en masse de styrène-butadiène, copolymère en masse de styrène-isoprène hydrogéné et copolymère en masse de styrène-butadiène hydrogéné.

8. Procédé de préparation d'une feuille absorbant les liquides, le procédé comprenant le transport d'une première couche de matière de base de tissu sous forme de feuille, la formation sur la première couche d'une deuxième couche d'une matière de base de tissu en vaporisant un polymère thermofusible sur la surface de la première couche.

9. Procédé selon la revendication 8, dans lequel le polymère thermofusible est au moins un polymère sélectionné dans un groupe consistant en polypropylène, polyéthylène, copolymère de polypropylène et de polyéthylène, copolymère en masse de styrène-isoprène, copolymère en masse de styrène-butadiène, copolymère en masse de styrène-isoprène hydrogéné et copolymère en masse de styrène-butadiène hydrogéné.

10. Article hygiénique comprenant une feuille absorbant les liquides selon l'une quelconque des revendications 1 à 4, sur un côté de laquelle est disposée une bande de matière de feuille absorbant les liquides.
